(19) Europäisches Patentamt
European Patent Office
Office européen des brevets

(11) EP 2 371 291 A1

(12) **EUROPÄISCHE PATENTANMELDUNG**

(43) Veröffentlichungstag:
05.10.2011 Patentblatt 2011/40

(51) Int Cl.:
*A61B 8/08* (2006.01)  *A61B 5/103* (2006.01)

(21) Anmeldenummer: 11158350.6

(22) Anmeldetag: 15.03.2011

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **31.03.2010 DE 102010003548**

(71) Anmelder: **Winkler, Dr. med., Roman**
**93077 Bad Abbach (DE)**

(72) Erfinder: **Winkler, Dr. med., Roman**
**93077 Bad Abbach (DE)**

(74) Vertreter: **nospat Patent- und Rechtsanwälte**
**Naefe Oberdorfer Schmidt**
**Isartorplatz 5**
**80331 München (DE)**

(54) **Bildgebendes Messverfahren und Vorrichtung**

(57) Die vorliegende Erfindung betrifft ein bildgebendes Messverfahren und entsprechende Vorrichtung zur Schaffung einer non-invasiven, frühzeitigen Diagnosemöglichkeit hinsichtlich einer Fehlbildung am Skelett und Gelenken. Ihr Anwendungsgebiet findet sich primär in der ambulanten Diagnostik und sekundär unterstützend in der stationären/operativen Therapie.

Um ein Verfahren und eine Vorrichtung zu schaffen, die völlig schmerzlos und frei von jeglicher Strahlenbelastung oder anderen gesundheitsgefährdenden Einflüssen eine Diagnose der vorstehend beispielhaft genannten Art bei verbesserter Zuverlässigkeit gestattet, wird vorgeschlagen, dass die mittels Auswertung von Ultraschallsignalen erhaltenen Messdaten mit Lagedaten kombiniert werden, die von einem mit der Ultraschallsende- und Empfangseinheit gekoppelten Lagesensor zeitlich parallel ausgegeben werden. Weiterbildungen und neuartige Anwendungen werden ebenfalls beschrieben.

Abb. 1.2:

EP 2 371 291 A1

**Beschreibung**

Die vorliegende Erfindung betrifft ein bildgebendes Messverfahren und entsprechende Vorrichtung zur Schaffung einer non-invasiven, frühzeitigen Diagnosemöglichkeit hinsichtlich einer Fehlbildung an Skelett und Gelenken. Im Rahmen der vorliegenden Erfindung werden ein bildgebendes Messverfahren sowie eine leicht bedienbare sowie schnell Ergebnisse liefernde Vorrichtung vorgeschlagen, die ihr Einsatzfeld in der ambulanten, wie auch der stationären Behandlung finden.

Aus der Medizintechnik sind bereits diverse Ansätze bekannt, Fehlbildungen am Skelett non-invasiv diagnostizieren zu können. Röntgen-, CT-und MRT-basierte Ansätze erweisen sich hierbei für eine Screening Untersuchung aufgrund der Strahlenbelastung und auch der hohen Kosten als nachteilhaft. Als eine kostengünstige und nicht strahlenbelastende Untersuchungsmethode bei nachweislich sehr geringer Belastung des Körpers steht in der Orthopädie die Ultraschalldiagnostik zur Verfügung. So ist in der Ultraschalldiagnostik unter dem Begriff der sog. "Hüftsonografie nach Graf" eine Untersuchung von Neugeborenen auf eine Hüftdysplasie bekannt, die zur schonenden und schnellen Erkennung derartiger Hüft-Fehlbildungen, die wohl die häufigste angeborene Erkrankung am Halte- und Bewegungsorgan darstellt, fakultativ eingesetzt wird.

Es ist Aufgabe der vorliegenden Erfindung, ein Verfahren und eine Vorrichtung zu schaffen, die völlig schmerzlos und frei von jeglicher Strahlenbelastung oder anderen die Gesundheit möglicherweise gefährdenden Einflüssen eine Diagnose der vorstehend beispielhaft genannten Art bei verbesserter Zuverlässigkeit gestattet.

Diese Aufgabe wird durch ein Verfahren mit den Merkmalen von Anspruch und eine entsprechende Vorrichtung gelöst. Erfindungsgemäß zeichnet sich ein neues Verfahren gegenüber bekannter Ultraschalldiagnostik im Bereich eines menschlichen Beckens durch die Verwendung einer Kopplung einer auch von Hand bewegbaren Ultraschallsende- und Empfangseinheit mit einem Lagesensor aus. Ein erfindungsgemäßes Messverfahren erhöht die Zuverlässigkeit einer nachfolgenden Diagnose dadurch, dass die mittels Auswertung von Ultraschall-Signalen erhaltenen Messdaten mit Lagedaten kombiniert und daher im Wesentlichen genau reproduzierbar sind. Diese Messungen sind untereinander vergleichbar, da sie gegenüber dem Schwerefeld der Erde durch den Lagesensor ausgerichtet durchgeführt werden. Über diverse Messungen z.B. an verschiedenen Patienten hinweg sind damit vergleichbare Ergebnisse erzielbar, durch die u.a. Abweichungen sicher erkannt werden können. Ein Hauptproblem der Ultraschalluntersuchung liegt dabei darin, dass es mangels eindeutiger Landmarken zu viele Freiheitsgrade gibt, um ein Hüftgelenk reproduzierbar darzustellen. Hier schafft nun der Lagesensor Abhilfe, der dabei vorzugsweise mit der Ultraschallsende- und Empfangseinheit starr gekoppelt ist und zusammen mit diesem eine mechanische Einheit bildet. Dabei wird vorteilhafterweise auf komplexe mechanische Apparaturen verzichtet, wie diese u.a. von Vorrichtungen zur "Hüftsonografie nach Graf" bekannt sind und dort zudem auch Fehlerquellen hinsichtlich der Genauigkeit des Messverfahrens darstellen können.

Vorteilhafte Weiterbildungen der Erfindung sind Gegenstand der jeweiligen Unteransprüche. Demnach bewirkt der Lagesensor in einer Ausführungsform der Erfindung eine senkrechte Ausrichtung der Ultraschallsende- und Empfangseinheit in einem Schallkopf selber oder es wird eine Abweichung von einer senkrechten Ausrichtung des Schallkopfes akustisch und/oder optisch angezeigt. Besonders bevorzugt ist, dass eine Abweichung des Schallkopfes von einer, vorab eingestellten z.B. senkrecht, Ausrichtung intern im Schallkopf ausgeregelt wird, insbesondere durch elektrische oder motorische Verstellung oder Lageänderung der Ultraschallsende- und Empfangseinheit.

In einer bevorzugten Ausführungsform der Erfindung werden aus den graphisch aufbereiteten Messdaten heraus charakteristische Größen zur Beurteilung einer Deformität unter Verwendung von Schablonen ermittelt. Hierzu können auch anstelle einer transparenten, auf ein UltraschallBild aufzulegenden Schablone mit Kurven, Hilfslinien und/ oder Winkelangaben insbesondere auf Bilderkennung basierende Algorithmen automatisch in einer Recheneinheit vorzugsweise gleich zur Ergebnisausgabe ablaufen.

Neben einer statischen Untersuchung wird in einer Ausführungsform der Erfindung mindestens ein weiterer Lagesensor zusätzlich an der zu untersuchenden Extremität angebracht, um die Extremität frei oder definiert gegenüber dem Schallkopf bewegen und die relative Winkelabweichung registrieren zu können. Mit einer Aufnahme über der Zeit ist damit eine zeitliche Lageänderung erfassbar.

In einer wesentlichen Weiterbildung der Erfindung werden parallel zu einer Lageänderung auch über der Zeit hin auftretende akustische Signale aufgenommen und ausgewertet. Derartige Signale treten über einen weiten Frequenzbereich auf, können aber in unmittelbarem Kontakt mit dem Körper und insbesondere nahe an einem Entstehungsort mit ausreichendem Störabstand aufgenommen werden. Die bei bestimmten Bewegungen entstehenden Geräusche der Gelenke werden durch mindestens einen mobilen Lage- und/oder Beschleunigungssensor aufgezeichnet. In einer hierauf aufbauenden Ausführungsform der Erfindung ist ein Patient zum Durchführen eines Messverfahrens nicht beispielsweise an eine Liege gebunden, der Patient kann sich mit diesen Sensoren vielmehr bewegen oder sogar sportlich aktiv sein. Eine Gelenkstellung, bei denen es im Laufe mobiler Untersuchungen zu einem pathologischen Geräusch im Gelenk gekommen war, kann dann bei einer späteren Ultraschalluntersuchung mittels der Vorrichtung rekonstruiert werden. Durch die Lagesensoren im Schallkopf und an mindestens einer Extremität kann eine reproduzierbare Ultraschall-Bildgebung einer Gelenkbewegung oder Gelenkstellung erfolgen, bei der es zur Entstehung von pathologischem Körperschall gekommen war.

Ohne Beschränkung des Einsatzbereiches sind ein erfindungsgemäßes Verfahren und eine entsprechende Vorrichtung damit u.a. einsetzbar für:

1. Ultraschall-Untersuchung von Erwachsenen zur kostengünstigen Screening-Untersuchung, bei der Ultraschalluntersuchung von Säuglingen, u.a. Hüftsonografie nach Graf;
2. Standardisierung der Vermessung von Gelenken mit Ultraschall;
3. Erzeugung von dreidimensionalem Knochenmodell unter Kopplung von Bildinformation mit Lageinformation der Gelenk- und der Transducereinheit.
4. Erfassung von im Gelenk entstehenden Körperschall mittels mobiler Lage- und Beschleunigungssensoren unter synchroner Aufzeichnung der Gelenkstellung;
5. Erzeugung eines dreidimensionalen Knochenmodells auf Grundlage einer hochauflösenden Kernspinuntersuchung zur Simulation von Gelenkbewegungen und denen dabei auftretenden Kräften im Gelenk.

Diese Aufzählung möglicher Einsatzbereiche ist nicht abschließend.

Nachfolgend werden Ausführungsbeispiele der Erfindung unter Darstellung weiterer Merkmale und die sich daraus ergebenden Vorteile unter Bezugnahme auf die Abbildungen der Zeichnung näher erläutert. In der Zeichnung zeigen in skizzierter Darstellung:

Abb.1.1:    Schematische Darstellung eines ovoid-förmigen Hüftkopfs.

Abb.1.2:    An einem Knochenmodell eingezeichnete Parameter, die zur Vermessung der Hüftkopfgeometrie verwendet werden.

Abb. 1.3.:   Ultraschallbild eines im Querschnitt aufgenommenen Hüftkopfs gemäß DEGUM-Richtlinie mit Einzeichnung des Diameters D und der Hilfslinie L1.

Abb. 1.4:   Schematische Darstellung analog der Abb.1.3 mit Einzeichnung der für die Vermessung des Diameters D notwendigen Hilfslinien.

Abb. 1.5:   Ultraschallbild einer gesunden Hüfte gemäß DEGUM-Richtlinie ventraler Längsschnitt.

Abb. 1.6:   Schematische Darstellung analog der Abb. 1.5 mit Einzeichnung der für die Vermessung der Hüftkopfgeometrie notwendigen Hilfslinien.

Abb. 1.7:   Ultraschallbild einer pathologischen Hüfte mit sonographisch erkennbarer CAM-Deformität und hochgradig vermindertem Hüftkopf-Schenkelhals-Offset.

Abb. 1.8:   Schematische Darstellung analog der Abb. 1.7 mit Einzeichnung der für die Vermessung der Hüftkopfgeometrie notwendigen Hilfslinien, im Unterschied zu Abb. 1.6 wird hier eine pathologische CAM-Deformität sowie ein HüftkopfSchenkelhals-Offset gezeigt.

Abb. 2.1:   Schematische Darstellung der Anordnung von zwei gravimetrischen Lagesensoren, wobei Lagesensor GS1 an einem Ultra- schalltransducer fixiert ist, Lagesensor GS2 ist zur Bestimmung der Gelenkstellung an der betreffenden Extremität angebracht.

Abb. 2.2:   In Analogie zu Abb. 2.1 mit nun veränderter Transducerposition und veränderter Extremitätenstellung.

Abb. 3:     Schematische Darstellung eines kabellosen, autonomen Lagesensors.

Abb. 4:     Schematische Darstellung einer UltraschallTransducereinheit mit Integration eines Lage- und Beschleunigungssensors mit automatischer Korrekturvorrichtung der Transducereinheit.

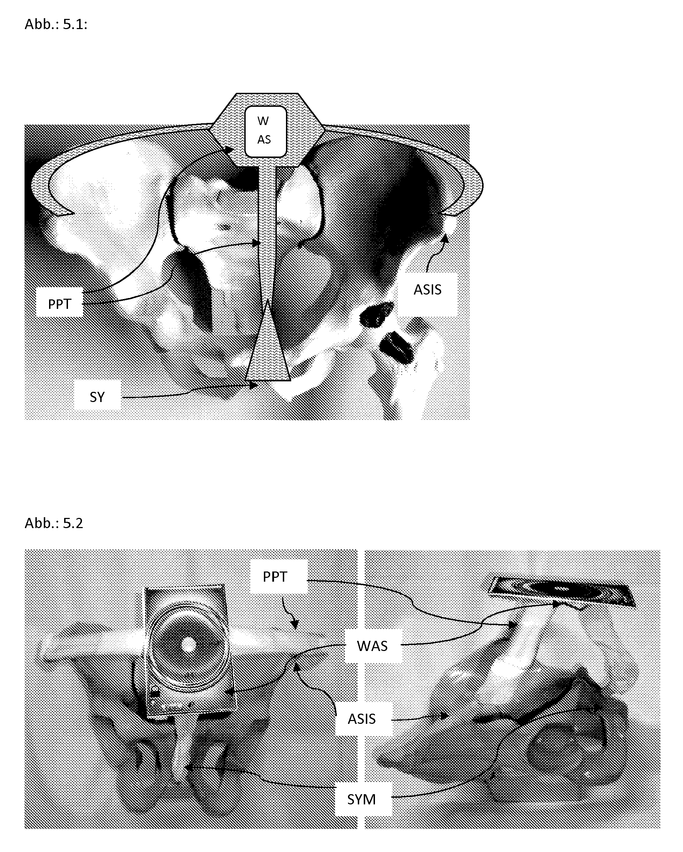

Abb. 5.1:   Schematische Darstellung der baulichen Einheit zur Bestimmung der vorderen Beckenebene.

Abb. 5.2:   Modell der baulichen Einheit zur Bestimmung der vorderen Beckenebene mit angebrachtem Lagesensor.

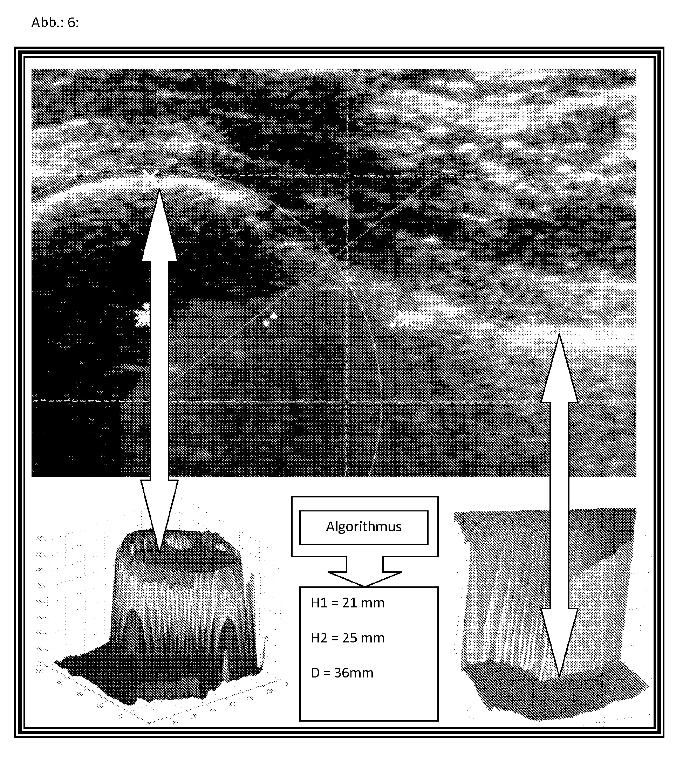

Abb. 6:     Schematische Darstellung des automatischen Analyse-Algorhythmus zur Vermessung von Ultraschallbildern, wodurch nach Konturerkennung vorgegebene Parameter autonom vermessen werden.

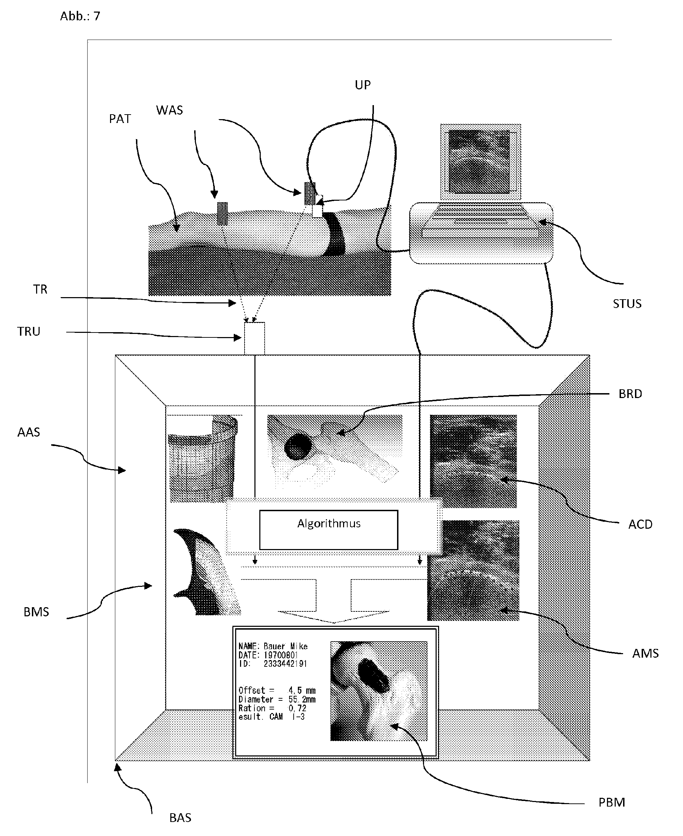

Abb. 7:     Schematische Darstellung des Analyse-Algorhythmus zur Erstellung eines 3D-Modells von Knochen auf Grundlage von Ultraschallbildern.

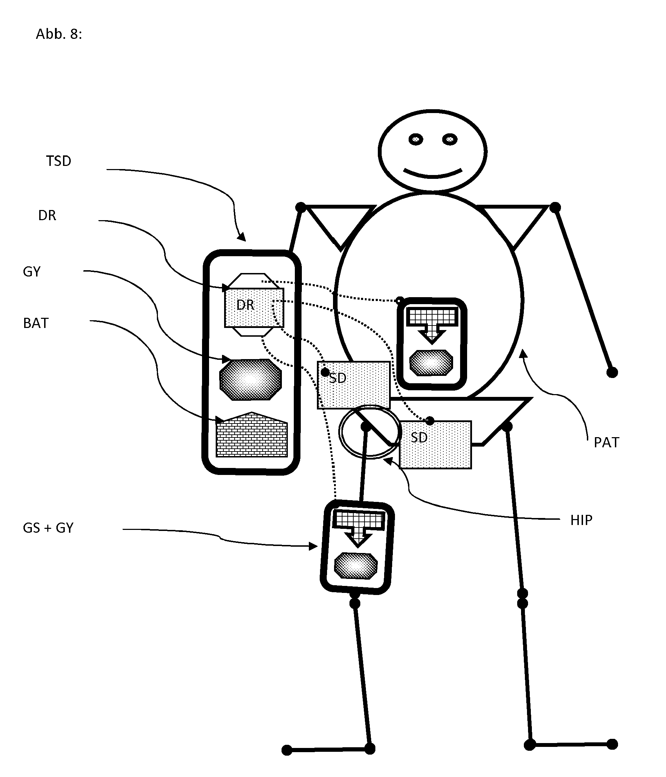

Abb.8:      Schematische Darstellung einer mobilen baulichen Einheit zur Erfassung von Körperschall unter Verwendung von zwei Schalldetektoren und zwei gravimetrischen Lagesensoren.

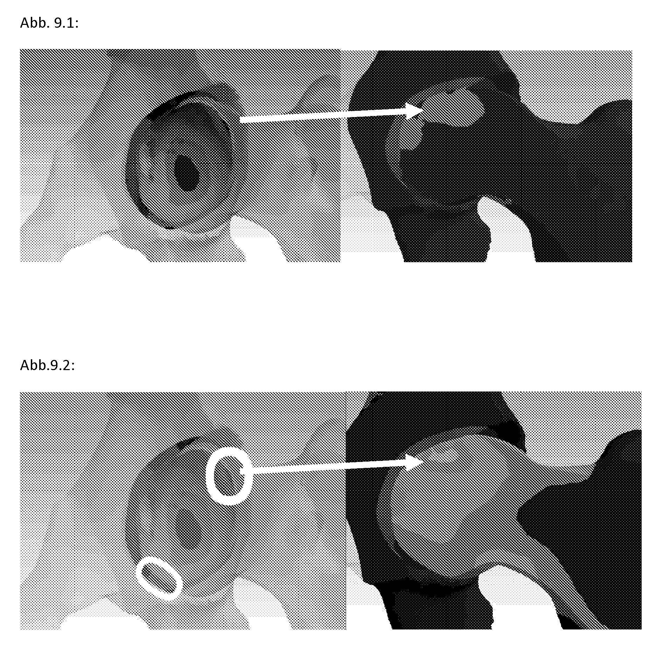

Abb. 9.1:   Schematisches Beispiel einer Bewegungsanalyse eines Finite-Elemente-Systems, wobei diese Abbildung eine normale Kräfteverteilung bei endgradiger Bewegung zeigt.

Abb. 9.2:   Schematisches Beispiel einer Bewegungsanalyse eines Finite-Elemente-Systems bei pathologischer Kräfteverteilung bei endgradiger Bewegung.

Zu Anspruch 1: Spezielles Diagnoseverfahren am Hüftgelenk

In der Humanmedizin wird die mechanische Kollision der knöchernen Anteile des Hüftgelenkes, also zwischen Schenkelhals bzw. Oberschenkelknochen, medizinisch Femur genannt, und der Hüftpfanne, medizinisch Acetabulum genannt, als femoroacetabuläres Impingement bezeichnet. Das femoroacetabuläre Impingement an sich ist keine Gelenkerkrankung, führt aber durch eine permanente mechanische Irritation über die Jahre hinweg zur Zerstörung des Hüftgelenkes. Bei einem pathologischen Kopf-Hals Übergang und damit unzureichender Taillierung des Schenkelhalses kommt es bei der Beugung des Gelenkes zu einem Einpressen des aspherischen Hüftkopfes in die Hüftpfanne. Diese minimale, aber sehr kräftige Aufdehnung des Acetabulums verursacht eine Delamination des Pfannenknorpels vom knöchernen Pfannengrund. Diese Art des Impingements wird als CAM-Impingement bezeichnet (Cam = englisch für Nockenwelle).

Da der vordere Anteil des Schenkelhalses nur durch wenig Gewebe bedeckt ist, kann die Region am Schenkelhals, welche die Schädigung im Gelenk hervorruft, grundsätzlich gut durch eine Ultraschalldiagnostik dargestellt werden. Bis heute gibt es kein einheitlich reproduzierbares Ultraschallmessverfahren durch welches die Hüfte objektivierbar vermessen wird.

Die Ursache für diese Formstörung am Schenkelhals ist nicht genau bekannt. Bekannt ist aber, dass es nach einer abgelaufenen Epiphysioloyse oder nach einer fehl-verheilten Schenkelhalsfraktur zu diesen Inkongruenzen zwischen Femur und Acetabulum kommen kann. Der Ursprung des Großteils der Formstörungen ist aktuell ebenfalls nicht bekannt. Die Formstörung liegt meistens in dem Sektor zwischen dem kopfwärts bzw. Position 12 Uhr und dem bauchwärts, d.h. Position 3 Uhr, gelegenem Abschnitt am Schenkelhals.

Als eines der ersten Symptome werden von den Patienten i.d.R. Leistenschmerzen bei Beugung und Innenrotation des Beines angegeben. Eine objektive Diagnose der Erkrankung kann derzeit nur durch eine Röntgenaufnahme oder durch eine Kernspintomographie erfolgen. Damit ist nach dem aktuellen Stand der Technik also eine Diagnose nur auf Basis sehr aufwendiger technischer Verfahren möglich, die u.a. auch belastend für den Patienten sein können, aber in jedem Fall mit vergleichsweise hohen Kosten verbunden sind.

Als technisches Hilfsmittel dient erfindungsgemäß mindestens ein Lagesensor, der die Orientierung des Schallkopfes im Gravitationsfeld der Erde bestimmt. Die Lage des Schallkopfes gegenüber der Horizontalen soll insbesondere in einer oder zwei Ebenen permanent bestimmt und akustisch/visuell ausgegeben und digital gespeichert werden. Diese technische Vorrichtung wird in einer Ausführungsform der Erfindung entweder bei bestehenden Ultraschallgeräten nachgerüstet oder in einem neu zu entwickelnden Schallkopf integriert. Die Kombination aus einem Lagesensor und einem Schallkopf ermöglicht entweder die Einstellung gewisser Schnittebenen, z.B. eine exakte Senkrechtstellung des Schallkopfes, oder die Vorgabe das Gelenk in verschiedenen Positionen exakt anzuschallen.

Als eine weitere präarthrotische Deformitäten des proximalen Femurs wird ein zu geringes Kopf-Hals-Offset (= OS) von weniger 7 mm angesehen. Auch diese Pathologie soll mit dem nachfolgend beschriebenen Messverfahren bestimmt werden.

Aufgrund von anatomischen und technischen Limitationen kann der Hüftkopf und der angrenzende Schenkelhals meist nicht auf einem Ultraschallbild dargestellt werden. Der zentrale Punkt dieser Erfindung ist die Vermessung des Hüftkopfes in zwei zueinander annähernd senkrecht stehenden Schnittbildern. Unter Berücksichtigung der Anatomie wird bei der Untersuchung der nicht einsehbare Anteil des Hüftkopfes mittels einer mathematischen Formel berechnet. Die Orientierung der Ultraschallschnittbilder erfolgt gemäß der DEGUM Richtlinie (Deutsche Gesellschaft für Ultraschallmedizin) und entspricht etwa dem ventralen Längsschnitt und dem ventralen Querschnitt der Hüfte.

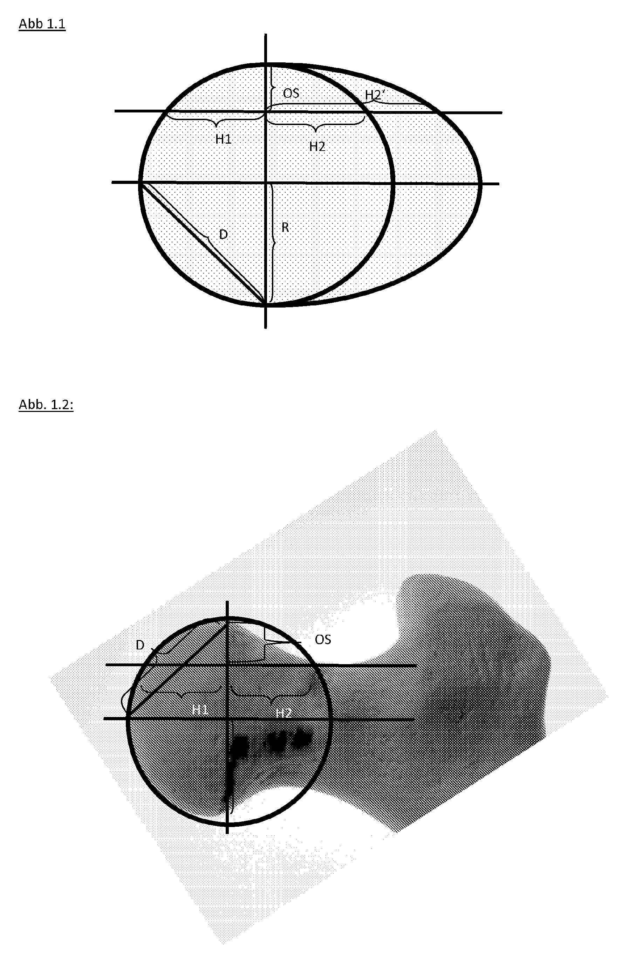

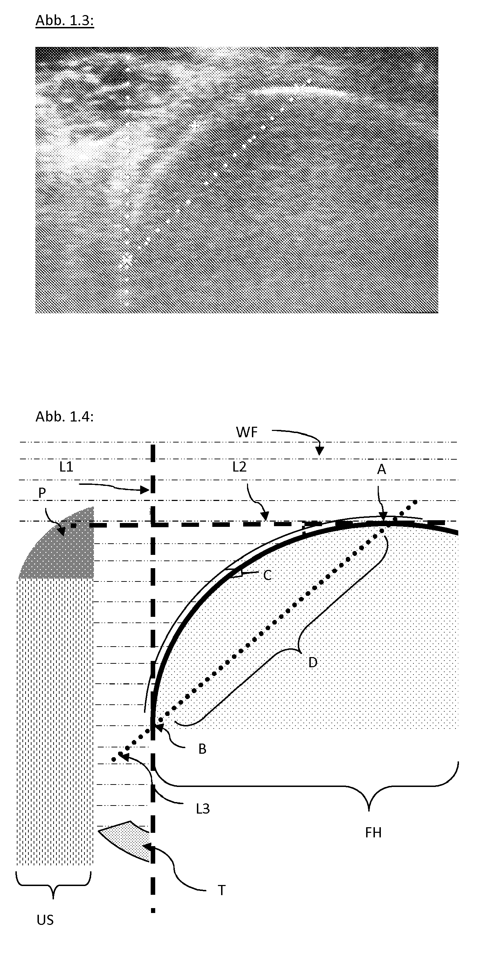

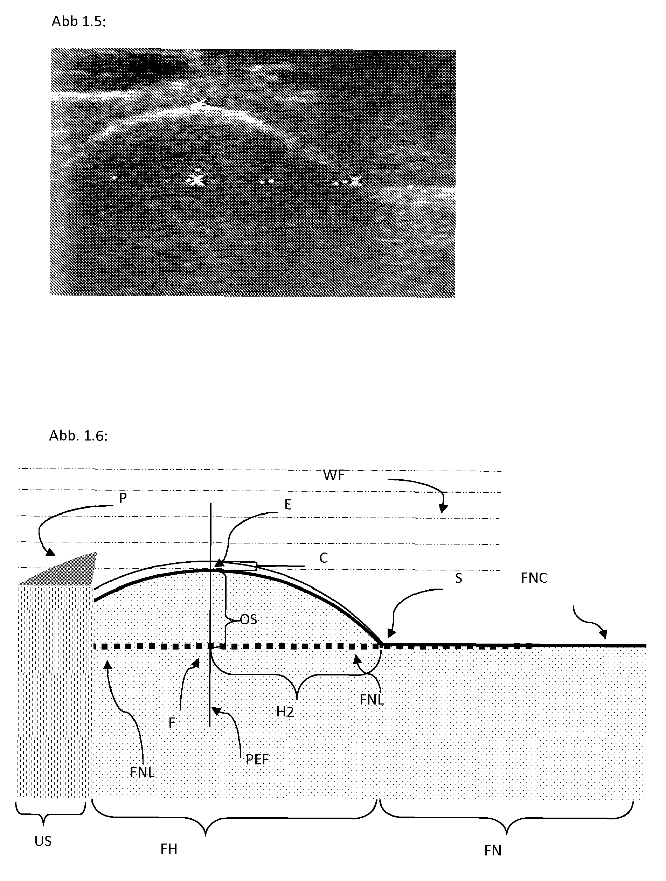

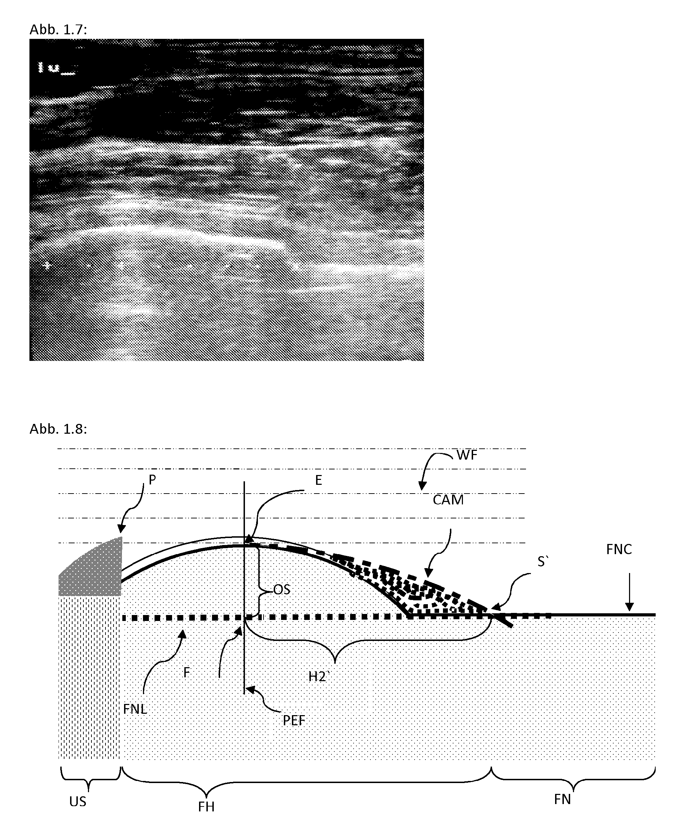

Figur 1.1 zeigt eine abstrakte zeichnerische Darstellung des oberen Teils eines Oberschenkelknochens mit Einblendung von Größen, die für Bestimmung eines als ideal kugelförmigen cranio-medialen Gelenkkopf-Anteils relevant sind als Hilfsskizze zur Berechnung einer relevanten Diagnose-Kenngröße aus in einem Sonogramm messbaren Parametern.

1.Teil: Bestimmung des Hüftkopfdurchmessers

Zur Vermessung des Hüftkopfdurchmessers (=FHD) liegt der Patient mit entspannten und geraden Beinen auf dem Rücken. Da das medio-kraniale Ende des Hüftkopfes meist in der Tiefe der Pfanne verborgen liegt, kann H1 nicht direkt gemessen werden. Man kann annehmen, dass der Hüftkopf entlang des Äquators und medio-kranial davon einer Kugel entspricht. Damit kann der Radius des Hüftkopfes entlang des Äquators, also nicht zwingend in der direkten Verlängerung von H2, gemessen werden. Aus dem Hüftkopfradius, dem gemessen Offset (=OS) und dem dazugehörigen H2, kann der korrespondierende Sekantenabschnitt H1 berechnet werden.

Etwa in 90° Stellung im Verlauf des zum Schenkelhals wird nun der höchste Punkt des Hüftkopfes aufgesucht. In dieser Einstellung wird der höchste Punkt des Hüftkopfes als Punkt A bezeichnet. Der Punkt A wird am rechten Bildrand positioniert, die konvexe Hüftkopfrundung fällt nach links hin ab. Durch Drehen und Verschieben des Schallkopfes wird der maximale Diameter des Hüftkopfes aufgesucht. Unter Beibehaltung von Punkt A wird durch Kippung des Schallkopfes ein Schallfenster aufgefunden, in dem der vordere Pfannenrand keinen Schallschatten erzeugt und ein Schallreflex in der Tiefe der Hüftpfanne sichtbar wird. Dieser Reflex wird als Tiefergewebereflex (=T) bezeichnet. Das Bild wird gespeichert und drei Hilfslinien werden eingezeichnet. Die Hilfslinie L1 verläuft senkrecht zur Schallebene (=WF), links tangential am Hüftkopf und rechts am Tiefengewebereflex (=T) vorbei. Die Hilfslinie L2 liegt parallel zur Schallebene und schneidet den Hüftkopf an seiner höchsten Stelle A tangential. Im 45° Winkel zu L1 und L2 wird nun die Hilfslinie L3 durch den Punkt A gelegt. Der Schnittpunkt von L3 mit L1 wird als B bezeichnet. Die Stecke zwischen den Punkten A und B wird als D bezeichnet und vermessen.

Alternativ zur Messung von D kann die Strecke vom Schnittpunkt der Hilfslinien L1 mit L2 und dem Punkt A als Radius R des Hüftkopfes gemessen werden. Alternativ dazu kann durch einen Analyse Algorithmus (Anspruchs 6) durch Formerkennung der Hüftkopfdurchmesser ermittelt werden.

2. Teil: Bestimmung des Kopf-Hals-Offsets

Zur Vermessung des Kopf-Hals-Offsets liegt der Patient mit entspannt gerade gestreckten Beinen auf dem Rücken. Der Schallkopf ist in ca. 45° zur Körperlängsachse ausgerichtet. In dieser Position sollen nun der ventralste und kranialste Punkt des Hüftkopfes aufgefunden werden. Dieser bildet im weiteren Verlauf der Untersuchung den Punkt E, durch den der Äquator des Hüftkopfes verläuft. Der Punkt E wird für die weitere Untersuchung an den linken Bildrand positioniert. Dies geschieht durch Verschieben des Schallkopfes nach lateral. Durch Drehung des äußeren (=lateralen) Anteils des Ultraschallkopfes um den Punkt E wird der Verlauf des Schenkelhalses (=FNC) eingestellt. Der Schallkopf wird dabei stets orthogonal zur Knochenoberfläche gehalten. Die exakte senkrechte Position erkennt man im Ultraschallbild an den deutlich helleren und schärferen Konturreflex des Knochens. Die Position des Schenkelhalses wird so eingestellt, dass zwischen dem höchsten Punkt des Hüftkopfes und dem höchsten Punkt des Schenkelhalses der geringste Höhenunterschied besteht. Dabei muss der Schenkelhals weiterhin maximal echoreich eingestellt bleiben, d.h. die Knochenoberfläche wird stets orthogonal angeschallt. Nun wird durch Absenken des lateralen Schallkopfanteils der Schenkelhals in eine horizontale Ebene gebracht, d.h. der Schenkelhals (=FNC) wird parallel zur Schallebene (=WF) eingestellt. Ist der Schenkelhals exakt parallel zur Wellenebene eingestellt, wird das Bild gespeichert. In dem gespeicherten Ultraschallbild wird eine horizontale Hilfslinie entlang des Verlaufes des Schenkelhalses eingezeichnet. Diese Hilfslinie wird als Schenkelhalsgrundlinie (=FNL) bezeichnet. Durch den Punkt E wird das Lot auf die Schenkelhalsgrundlinie (=FNL) gefällt. Der Schnittpunkt des Lotes mit der Schenkelhalsgrundlinie wird als Fußpunkt (=F) bezeichnet. Im Ultraschallbild wird der Abstand zwischen E und F gemessen. Dieser Wert entspricht dem Kopf-Hals-Offset des Femurs (=OS).

3. Teil: Vermessung des lateralen Hüftkopfes H2

Gemessen wird der Abstand vom Fußpunkt (=F) zum Scheitelpunkt (=S). Der Scheitelpunkt (=S) ist die Stelle, an dem die Kontur des Femurhalskortikalis die Schenkelhalsgrundlinie nach ventral verlässt. Der Abstand zwischen F und S wird als lateraler Sekantenabschnitt (=H2) bezeichnet. Es bietet sich an, die Messung von H2 in den identischen Schnittbildern, in denen das Offset bestimmt wurde, durchzuführen. Man kann aber auch eine weitere Einstellung aufsuchen, in der sich sonographisch eine deutliche Kopf-Hals-Übergangsstörung im Sinne einer ausgeprägten CAM-Deformität darstellt. In diesem Schnitt ist erneut das Kopf-Hals-Offset zu bestimmen. Die Positionsbeschreibung der Pathologie erfolgt mit Hilfe eines Lagesensors (Anspruch 3) und wird als Untersuchung in Anspruch 2 beschrieben.

4. Teil: Berechnung der Hüftkopfgeometrie

Es wird angenommen, dass die Pathologie des Hüftkopfes entlang nur einer Achse besteht, und zwar im Verlauf des Schenkelhalses lateral des Äquators. So entspricht der Hüftkopf beim Cam-Impingement einem Ovoid. Auf der medialen Seite gleicht der Hüftkopf einer Kugel, auf der lateralen Seite einem ellipsoiden Körper. An dem Hüftkopf sollen die Abschnitte H1 und H2 einer Sekante des Hüftkopfes bestimmt werden. Der Abschnitt H1 liegt kranio-medial des Äquators, H2 liegt in der Verlängerung von H1 im Verlauf des Schenkelhalses lateral des Äquators. Für den Fall, dass der Hüftkopf einer Kugel entspricht, ist H1 gleich mit H2. Hat der laterale Anteil des Hüftkopfes einen größeren Radius, dann ist H2 größer als H1. Dies ist der Fall beim CAM-Impingement. Aus den Werten OS, H2 und D kann nun der Wert H1 berechnet werden. Wird R gemessen so gilt D=R*√2'

Berechnung von H1:

$$H1 = \sqrt{(\sqrt{2} * OS * D - OS^2)} = \sqrt{OS(\sqrt{2} * D - OS)}$$

5. Teil: Klinische Interpretation der Messwerte - Bestimmung der Exzentrizität

Ziel der Bestimmung der Exzentrizität ist, festzustellen ob der Hüftkopf medial des Hüftkopfäquators den identischen Radius wie lateral des Äquators hat. Jegliche weitere mathematische Beschreibungen der Exzentrizität (lineare, numerische etc.) des Hüftkopfes unter Berücksichtigung des Hüftkopf-Schenkelhals Offsets sind ebenfalls Gegenstand des Patentes.

Um H1 und H2 miteinander vergleichen zu können, muss bei H1 noch die Knorpelschichtdicke (=C) des Femurkopfes berücksichtigt werden. Dieser Knorpelanteil fehlt physiologischerweise auf der Seite von H2 völlig. Der Wert von C kann entweder im Ultraschallbild gemessen oder pauschal (physiologische Knorpelschichtdicke) mit 2 mm angenommen werden. Wenn H1 + C = H2, dann ist der Femurkopf eine Kugel, das Ergebnis ist normwertig. Wenn H1 + C < H2 dann ist der Femurkopf keine Kugel, es besteht der Verdacht einer Kopf-Hals-Übergangsstörung im Sinne eines Nockenwellen (= CAM) Impingements.
Jegliche weitere klinische Beurteilungen auf Grundlade von mathematischen Algorithmen (Beschreibungen der lineare, numerischen Exzentrizität) oder Angabe des pathologischem Volumens unter Berücksichtigung des Hüftkopf-Schenkelhals Offsets sind ebenfalls auf dieser Basis möglich.

Weitere diagnostische und therapeutische Schritte sollten in einer entsprechend ausgerichteten Klinik eingeleitet werden. Zum einen kann durch eine radiologische Untersuchung der Befund bestätigt werden, zum anderen kann durch einen form-korrigierendne Eingriff am Schenkelhals eine vorzeitiger Gelenkverschleiß verhindert werden.

Zu Anspruch 2: Standardisierung von Ultraschalluntersuchungen durch Verwendung von mindestens zwei gravimetrischen Lagesensoren

Mit dieser technischen Vorrichtung ist die Ultraschalluntersuchung zur Vermessung von Gelenken einfach und kostengünstig zu standardisieren. Eine quantitative Vermessung von Gelenken ist damit interindividuell reproduzierbar möglich. Damit können Gelenkmorphologien einheitlich beschrieben und direkt miteinander verglichen werden.

Das Verfahren beruht auf der Dokumentation der Lage des Schallkopfes und in Relation zur Stellung des zu untersuchenden Gelenkes. Die Ultraschalluntersuchung kann wie gewohnt dynamisch erfolgen, d.h. der Untersucher kann wie gewohnt den Schallkopf und die zu untersuchende Extremität frei gegeneinander bewegen. Ein absolutes Positionssystem zur Bestimmung der exakten räumlichen Position der Sensoren, im Raum und deren exakte Stellung zueinander ist nicht nötig. Damit unterscheidet sich diese Erfindung von anderen Verfahren, die eine visuelle oder elektromagnetische Positionsbestimmung verwenden. Auf diese erweiterte Technik wird bei dem hier vorgestellten Verfahren verzichtet.

Ablauf der Untersuchung

Ein am Schallkopf angebrachter gravimetrischer Lagesensor (WAS) vermisst permanent die Stellung des Schallkopfes im Bezug zum Schwerefelde der Erde. Durch einen weiteren Lagesensor, der an der zu untersuchenden Extremität angebracht wird, wird synchron die Stellung des Gelenks in Relation zum Schwerefeld der Erde erfasst. Zu Beginn der Messung wird an der, dem zu untersuchenden Gelenk angrenzenden Extremität, ein Lagesensor angebracht. Der Patient wird in einer standardisierten Ausgangsposition, der sog. Neutralposition gelagert. Diese Neutralposition ist abhängig von dem zu untersuchenden Gelenk und variiert je nach klinischer Fragestellung. In der Neutralposition wird der Lagesensor auf null kalibriert. Der Schallkopf wird ebenfalls in Neutralposition auf null kalibriert. Die Ultraschalluntersuchung erfolgt stets in exakter orthogonaler Ausrichtung des Schallkopfes zur Knochenoberfläche. Die orthogonale Ausrichtung zeigt sich im Ultraschallbild durch einen sehr scharfer du deutlichen Schallreflex an der Knochenkortikalis und durch das Auftreten von sog. Reverberationsartefakte.

Bei der Ultraschalluntersuchung kann nun der Untersucher den Schallkopf und die Extremität frei bewegen. Ist der Befund lokalisiert, wird der Schallkopf über die gesamte Knochenoberfläche orthogonal ausgerichtet. Aus der Lagedifferenz der beiden Sensoren zueinander, wird die Position des dargestellten Befunds an der untersuchten Extremität berechnet. Die Ausgabe der Winkeldifferenz kann auf einem weiteren Gerät erfolgen oder wird direkt im Ultraschallgerät angezeigt.
Eine weitere Möglichkeit ist die graphische Positionsdarstellung an einem Knochenmodell wie in Anspruch 6 beschrieben. Je nach Gelenk erfolgt die Beschreibung der Lokalisation gemäß einer speziellen Nomenklatur. Bei speziellen Fragestellungen können beide Gelenkpartner mit einem Lagesensor versehen werden. Damit lässt sich, zusätzlich zur Schallkopfeinstellung, die gesamte Gelenkstellung dokumentieren. Damit kommen 3 Lagesensoren zum Einsatz.

Anwendungsbeispiel: Hüftgelenk / Schenkelhals

Als Beispiel soll die Vermessung des Schenkelhalses, wie im Anspruch 1 beschrieben, unter Dokumentation der Position am Schenkelhals erfolgen. Der Patient wird in Rückenlage auf der Untersuchungsliege gelagert. Ein Lagesensor wird am Knie der zu untersuchenden Seite angebracht. Bei gestrecktem Bein und senkrecht ausgerichtetem Fuß oder senkrecht hängendem Unterschenkel, wird in dieser Neutralposition der Lagesensor auf null kalibriert. Der Schallkopf wird parallel zum Boden ausgerichtet und der Lagesensor ebenfalls auf null kalibriert. Die Messung wird wie im Anspruch 1 beschrieben durchgeführt. Zum Auffinden der maximalen CAM-Deformität wird dynamisch die maximale Deformität am Schenkelhals aufgesucht. In der Position der maximalen Kopf-Hals-Deformität wird die Messung wie in Anspruch 1 beschrieben durchgeführt. Aus den erfassten Winkeln zwischen Schallkopf und der Stellung des Oberschenkels kann die Position der CAM-Deformität am Schenkelhals berechnet werden. Die Kenntnis dieser Position ist für die Beurteilung der Pathologie nötig und hilfreich bei der Planung einer Operation.

Beispiel einer Messung:

Stehen die beiden Sensoren senkrecht zueinander, so befindet sich der aufgefundene Befund bei 3 Uhr am Schenkelhals, also exakt in ventraler Position. Bei 30° Neigung des Schallkopfes nach cranial befindet sich die erfasste Schnittebene in 2 Uhr Position am Schenkelhals.

Beispiel Hüftgelenk bei Hüftdyspalsie

Unter der Verwendung eines Lagesensor zur Bestimmung der vorderen Beckenebene (Anspruch 4) kann durch eine Ultraschalluntersuchung die Überdachung des Hüftkopfes durch die Hüftpfanne gemessen werden. Bei der Untersuchung liegt der Patient auf dem Rücken. Der Untersucher stellt nun von ventral nach lateral verlaufend das Hüftgelenk dar. Unter orthogonaler Anschallung wird der Pfannenrand und der Hüftkopf eingestellt. Durch die Verkippung des Schallkopfes gegenüber der vorderen Beckenebene wird in unterschiedlichen Positionen so der Pfannenrand vermessen. Die Kopfüberdachung wird umlaufend um das Gelenk in vorgegebenen Positionen (15° Schritten) vermessen. Zur Darstellung der seitlichen Überdachung ist es vorteilhaft den Patienten, bei unverändertem Beckenlagesensor, in eine Seitenlage zu bringen und die Untersuchung dann vorzusetzen. Die Differenz zwischen der Lade des Schallkopfes und der vorderen Beckenebene wird erfasst. Aus den erfassten Daten wird die Hüftkopfüberdachung berechnet. Sämtliche Verfahren zur Beurteilung der Hüftkopfüberdachung z.B. mathematische Berechung der Überdachung in Prozent oder graphische Auswertung sind dem Fachmann bekannt und werden daher hin nicht weiter erläutert.

Beispiel Schultergelenk / Rotatorenmanschette

In Rückenlage des Patienten wird an der Innenseite des Unterarms der Lagesensor angebracht und der Unterarm senkrecht zur Untersuchungsliege aufgestellt. In dieser Position wird der Sensor auf null gesetzt. Der Schallkopf wird parallel zum Boden ausgerichtet und auf null gesetzt. Der Untersucher kann nun die Schulter von allen Seiten schallen, wobei durch Bewegung des Unterarms verschiedene Anteile der Rotatorenmanschette eingestellt werden können. Durch die Stellung von Unterarm und Schallkopf zueinander und bei direktem orthogonalen Anschallen, kann exakt die Position einer Pathologie z.B. einer Rotatorenmanschettenruptur oder eine Lage eines Kalkdepots ermittelt werden. Diese Erkenntnis ist bei der Planung und Ausführung einer Operation sehr hilfreich.

Zu Anspruch 3: gravimetrischer Lagesensor mit drahtloser Datenübertragung.

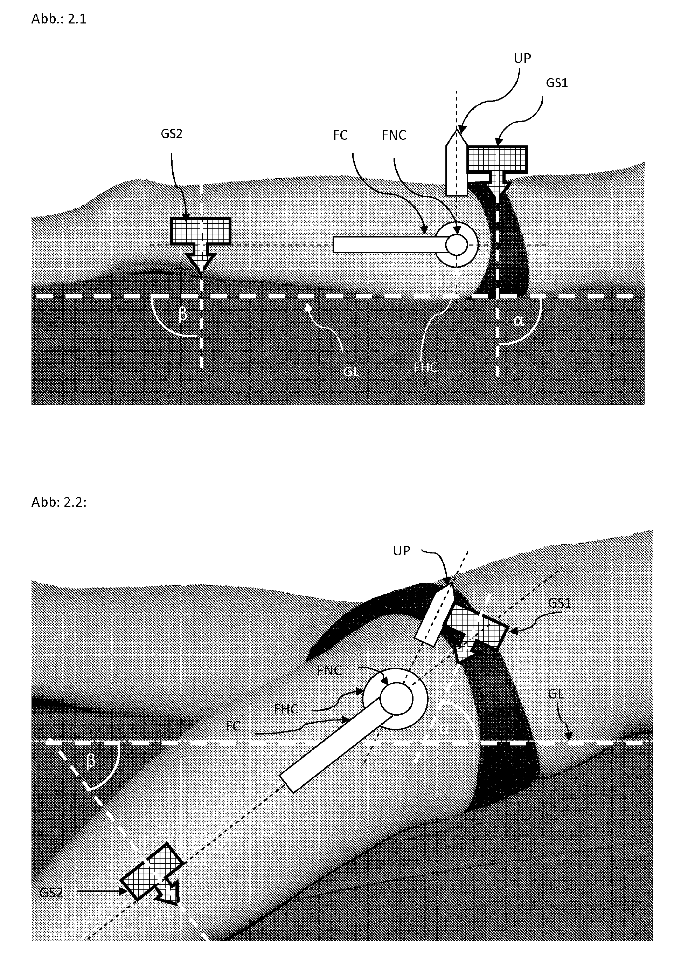

Für die Lagebestimmung von medizinischen Gerätschaften z.B. Ulltraschalltransducer, Punktionskanülen, sterile Zeigeinstrumente wird ein autonomer gravimetrischer Lagesensor mit drahtloser Datenübermittlungseinheit vorgeschlagen, hier als WAS (= wireless angulation sensor) bezeichnet Abb. 2.1. Technisch erfolgt die Lagebestimmung im Schwerefeld der Erde durch einen Beschleunigungssensor (GS) und einen Winkelbeschleunigungssensor bzw. Gyroskope (GY). Durch den Beschleunigungssensor wird die Winkelabweichung gegenüber dem Gravitationsfeld der Erde bestimmt, der Winkelbeschleunigungssensor wird für eine Kompensation von Bewegungsartefakten verwendet. Die aktuellen Beschleunigungsdaten werden drahtlos (TRU) an ein weitere Geräte übertragen. Ein WAS kann mit einem Strom-Akku (BAT)versehen sein. Durch die kompakte Bauart können diese WAS sterile verpackt und so leicht bei Operation verwendet werden. Die Kalibrierung des Lagesensors erfolgt in Neutralposition - z.B. auf einem Untersuchungstisch oder OP-Tisch.
Alternativ dazu, können die WAS auch durch die in Transponder Technik verwendeten Energie Übertragungstechniken versehen werden. Durch diese Technik sind kostengünstige einmalig verwendbare Produkte herzustellen.
Ein WAS kann an ein Operationsinstrument angebracht und/oder an der Extremität eines Patienten angebracht werden. Damit kann wären der Operation die Lage eines Instruments gegenüber der Unterlage oder gegenüber des Patient bestimmt werden kann.
Eine mögliche Anwendung wäre die Bestimmung der Anteversion bei der Implantation einer Hüftpfanne.

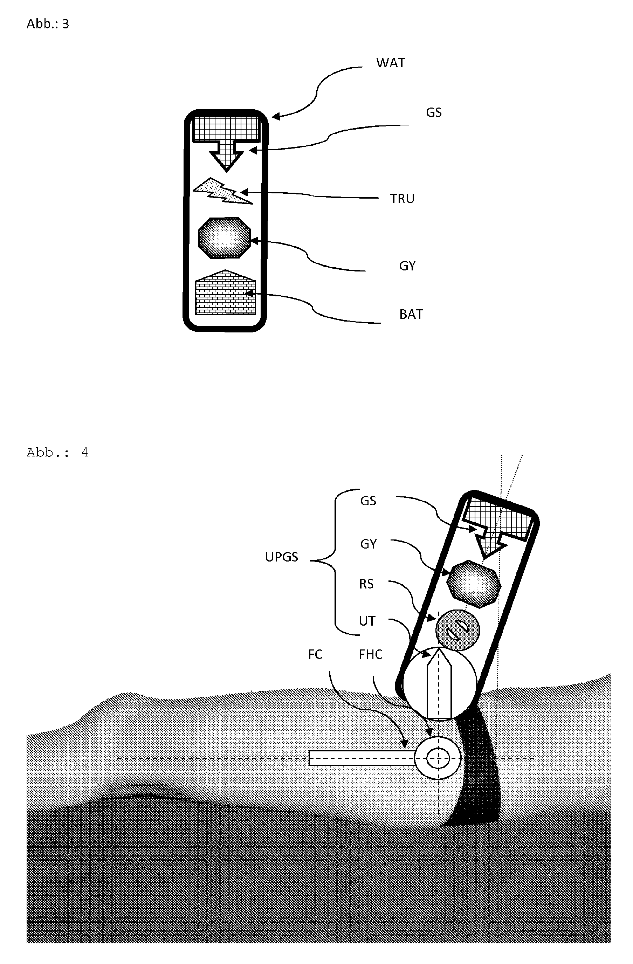

<u>Zu Anspruch 4: Kombination aus einem Lagesensor und einer automatischen Lageregelung der Transducer Einheit in einer geschlossenen Schallkopfeinheit (vgl. Abb. 3)</u>

Durch einen baulichen Zusammenschluss von einem Beschleunigungssensor (GS) z.B. wie in Anspruch 2 beschrieben und einer in seiner Lage automatisch veränderbaren Transducereinheit (UT) wird ein Schallkopf (UPGS) hergestellt, der automatisch einen gewünschten Anschallwinkel verändern kann. Damit kann der Untersucher sich ausschließlich auf die Einstellung eines optimalen Bildes konzentrieren, die Einstellung der gewünschten Lage erfolgt durch die automatische Lagekorrektur (RS). Die Lagekorrektur kann elektromechanisch oder nur elektronisch erfolgen.

Zur Kompensation von raschen Beschleunigungswerten, die bei Bewegungen des Schallkopfes erfolgen, wird durch einen Winkelbeschleunigungssensor (Gyroskop) die Winkelbeschleunigung erfasst und damit der Ausschlag der Transducer Einheit gedämpft. Allein die Auslenkung des Schallkopfes gegenüber dem Gravitationsfeld der Erde wird als Information verarbeitet und der Schallkopf entsprechend ausgerichtet.

Anwendung beim Säuglingsschall

Eine mögliche Anwendung ist der oben erwähnte Säuglingsschall nach Graf, bei dem eine senkrechte Anschallung der Hüfte gefordert wird. Eine bereits existierende, rein mechanische Lösung wird von Herrn Graf beschrieben. Der sog. "Sono-Guide" ist ein mechanischer Führungsarm, der den Schallkopf stets senkrecht ausrichtet. Die hier vorgeschlagene Erfindung gewährleistet eine automatische Ausrichtung des Ultraschalltransducers in die geforderte senkrechte Position. Der Untersucher kann den Schallkopf frei bewegen und wird nicht durch die mechanische Kopplung des Führungsarms in seiner Bewegung limitiert.
Eine weitere Anwendung findet sich bei der Dokumentation und einer Wiedereinstellung von pathologischen Befunden. Ist die exakte Position der Pathologie bekannt, so kann ein zweiter Untersucher diese Position, nach Einstellung der Position am Ultraschallgerät, exakt reproduzieren. Bei der Dokumentation von prä- und postoperativen Befunden wird das System bei einer Verlaufskontrolle zum exakten Einstellen eines Befundes verwendet.

<u>Zu Anspruch 5: Bauliche Einheit zur Bestimmung der vorderen Beckenebene</u>

Für die Bestimmung der Lage des Beckens eines Patienten wird die vordere Beckenebene bestimmt. Hierfür wird eine Beckenlagemessgerät (=PPT) verwendet. Das Beckenlagegerät kann baulich mit einem autonomen Lagesensor gemäß der in Anspruch 3 beschriebene Einheit versehen werden oder ist mit einem fest verbauten Beschleunigungssensor und Winkelbeschleunigungssenor versehen. Die Datenübertragung erfolgt entweder über ein Kabel oder per Funk. Der PPT wird beidseits an der Spina iliaca anterior superior und der Mitte über der Symphyse angebracht. Die Fixierung erfolgt entweder direkt an der Haut, oder bei einem narkotisierten Patienten direkt am Knochen. Der PPT hat drei Ausleger, die T-förmig angeordnet sind. In der Mitte zwischen den Auslegern befindet sich der Beschleunigungssensor. Der PPT kann auch aus einem sterilisierbarem Material bestehen und kann so bei Operationen verwendet werden. Der PPT kann zusätzlich über eine fluoroskopische Röntgenaufnahme auf die aktuelle Beckenposition bezogen werden. Im Verlauf einer Operation steht damit eine permanente Beckenpositionsangabe zur Verfügung. Des Weiteren kann das Beckenlagemessgerät durch die Verwendung von Referenzsternen in ein bestehendes stereoskopische Navigationssystem (z.B. Firma BrainLab) eingebunden werden. Dadurch lässt sich die Genauigkeit des Navigationssystems verbessern.

Anwendungsbeispiel Beckenlage Messgerät:

Ultraschalluntersuchung zur Bestimmung einer Hüftpfannendysplasie
Die Lagedaten vom Schallkopf, der Extremität und des Beckens werden in der zentralen Rechnereinheit verarbeitet. Aus den Daten vom Schallkopf und der Beckenposition kann die Pfannenüberdachung berechnet werden. Es werden verschiedene Messungen in unterschiedlichen Positionen am Beckenrand durchgeführt. Damit kann die gesamte Pfannenüberdachung bestimmt werden. Dieser Wert ist zur Beurteilung nötig ob eine normale Überdachung der Hüfte vorliegt oder ob die Überdachung zu gering ist, was eine Hüftdysplasie entspricht. Liegt eine Überüberdachung vor so spricht man von einem Beißzangenimpingement (= Pinzer-Impingement).

Anwendungsbeispiel Beckenlagemessgerät bei Operationen

Zur Anwendung kommt ein PPT mit einem Beschleunigungssensor z.B. bei der Implantation einer Hüftendoprothese. Durch das Beckenlagemessgerät (PPT) wird die Lage des Beckens bestimmt. Kommt es während der Operation zu Bewegungen des Patienten zeigt das Beckenlagegerät unverändert die Stellung des Beckens an. Wird ein zweiter Lagesensor (WAS) an einem Operationsinstrument befestigt, so kann die Winkeldifferenz zwischen beiden Instrumenten bestimmt werden. Dies gibt dem Operateur die relative Position des Instrumentes gegenüber dem Becken an. Damit erhält der Operateur stets die Information über die Position z.B. einer Fräse oder die zu implantierende Hüftpfanne. Durch dieses kostengünstige Hilfsmittel kann die Implantation von Hüftprothesen deutlich verbessert werden.

Zu Anspruch 6 Rechnerunterstütze Datenanalyse von Ultraschallbildern (i.V.m. Abb.: 5)

Eine erweiterte Möglichkeit besteht in der Bereitstellung einer Zusatzeinheit für bestehende Ultraschallgeräte. Dieser Nachrüstsatz soll die von dem Ultraschallgerät ausgegebenen Bilder in Abhängigkeit von der räumlichen Lage speichern und automatisch und autonom analysieren. Die Analyseeinheit führt selbstständig Messungen in den Schnittbildern durch. Ein zeitaufwendiges manuelles Vermessen wie bei Anspruch 1 und 2 beschrieben, entfällt. Mit dieser Zusatzeinheit können entweder Ultraschallgeräte ohne großen technischen Aufwand nachgerüstet werden, oder neue Geräte werden damit zukünftig ausgestattet werden.

Der Untersuchungsablauf wird beschleunig und die Objektivität steigt, was sich in einer noch gesteigerten Akzeptanz der Anwendung einer derartigen Vorrichtung niederschlagen wird. Das Messverfahren ist dadurch gekennzeichnet, dass unter Verwendung von Bildanalysesoftware und von digitalen Schablonen, aus den Ultraschallbildern heraus charakteristische Größen zur Beurteilung einer Deformität autonom ermittelt werden. Wobei die auf Bilderkennung basierende Algorithmen automatisch in einer Recheneinheit ablaufen und die Messdaten ausgeben. Eine mögliche autonome Analyseeinheit hier als HIP-MON bezeichnet, erfasst synchron die Daten der Beschleunigungssensoren (z.B. WAS) und die Bilddaten des Ultraschallgerätes. Durch den Untersucher müssen gewisse, jeweils für ein Gelenk typische Schnittebenen eingestellt werden. Die exakte Einstellung des Schallkopfes wird entweder durch den Untersucher vorgenommen oder erfolgt wie in Anspruch 4 beschrieben automatisch durch das Analysesystem. Zur Bildanalyse kommen verschiedene Algorithmen, wie zum Beispiel spezielle Konturenerkennungs- Algorithmen zum Einsatz (ACD). In den erkannten Konturen werden z.B. die wie in Anspruch 1 beschrieben Messungen autonom durchgeführt. Die Messdaten werden entweder gesondert ausgegeben oder an das Ultraschallgerät übergeben und im Ultraschallbild mit angezeigt.

Zu Anspruch 7: Erstellung eines dreidimensionalen Knochenmodells aufgrund von Ultraschallbildern

Entgegen gängiger 3D Ultraschallbildgebung wird nicht das aufgezeichnete Ultraschallbild als drei dimensionaler Datensatz gespeichert und analysiert, vielmehr wird aus den in Anspruch 6 beschriebenen Messungen ein Knochenmodell an die Messungen angepasst. Diese Software wird als Formmodellierungssystem bezeichnet (BMS). Die Grundlage ist ein für jeden Knochen und jedes Gelenk zugrunde liegendes mathematisches Computermodell hier als Referenzdatenbank für Knochengeometrie = BRD bezeichnet.

Unter der Kenntnis des zu untersuchenden Gelenkes werden relevante Messungen erstellt. Die Zuordnung der geschallten Struktur zur Position im Modell erfolgt durch einen gravimetrischen Lagesensor am Ulraschalltransducer und an der zu untersuchenden Extremität.

Beispiel:

Erstellung eins Schenkelhalsprofiles

Die Ultraschalluntersuchung erfolgt wie in Anspruch 1 und Anspruch 2 beschrieben, unter der Verwendung von Beschleunigungssensoren wie in Anspruch 3 beschrieben. Zur Bestimmung der vorderen Beckenebene wird ein Beckenlagemessgerät verwendet, wie in Anspruch 5 beschrieben. Damit stehen dem Bildanalysesystem die gravimetrischen Lagedaten der Transducereinheit und den Gelenk anschließenden Knochen (Becken und Femur) zur Analyse zur Verfügung. Durch die in Anspruch 6 beschriebene Analysesoftware werden vorgegebene Kenngrößen ermittelt. Diese Kenngrößen werden an das Formmodellierungsystem übergeben. Das zugrundeliegende Knochenmodell wird nun an die gemessenen Daten angepasst und daraus ein 3D Modell erstellt.

Zu Anspruch 8: Aufzeichung von Gelenkgeräuschen durch eine mobile Einheit unter Dokumentation der Gelenkstellung

Als Geräuschdetektoren kommen Mikrophone oder spezielle Beschleunigungssensoren zum Aufzeichnen von Körperschall zum Einsatz. Der technische Fortschritt der vergangenen Jahre hat schon im Bereich der Konsumelektronik so hoch spezialisierte Geräte mit zahlreichen Sensoren und integrierten drahtlosen Kommunikationsmitteln hervorgebracht, dass die Funktion eines erfindungsgemäßen Lagesensors beispielsweise durch die Technik eines Smartphones, wie z.B. iPhone®, oder einer Eingabevorrichtung für eine Spielekonsole, wie z.B. Wii®, realisiert werden kann. Derartige Geräte sind schon heute dazu in der Lage, zeitlich parallel zu einer Lage oder Lageänderung auch akustische Signale aufzunehmen und für die spätere Verarbeitung zu speichern oder zumindest für eine weitergehende Auswertung schnell an eine Auswertungseinheit zu übermitteln.

Die Aufzeichnung der Körperschallemissionen erfolgt in jedem Fall synchron mit Lagebestimmungen und Lageänderungen der an das zu untersuchende Gelenk anschließende Extremität. Eine ausreichende Versorgung mit elektrischer Energie wird über Akkumulatoren bzw. Speicherzellen mit heute verfügbaren Mitteln gut realisierbar. Die Daten werden stationär ausgewertet. Die Stellung der der Extremitäten, bei der es zu einem pathologischem Gelenkgeräusch gekommen war anschließend für eine Ultraschalluntersuchung rekonstruiert werden. So können gezielt pathologische Veränderungen die spezielle Geräusche erzeugen können so aufgedeckt werden. Für eine genaue Lokalisation des Entstehungsortes von Gelenkgeräuschen können auch mehr als ein Körperschall-Detektor zum Einsatz kommen. Der Entstehungsort kann durch mathematische Berechnung lokalisiert werden.

<u>Zu Anspruch 9: Gelenksimulation zur Analyse pathologischer Gelenkkonstellationen.</u>

In den letzten Jahren hat sich das Verständnis wie es zu einem Gelenkverschleiß kommt entscheidend erweitert. Durch die Erkenntnis, dass eine geringe Formstörung am Schenkelhals zu einer Schädigung des Hüftgelenks führt, ist eine dynamische Betrachtung des Hüftgelenkes von Interesse geworden. Welche anatomischen Parameter und welche Abweichungen davon zu einem Verschleiß des Hüftgelenkes führen ist nicht bekannt. Ziel ist hier eine individuelle dynamische Analyse einer Patientenhüfte, durch die Erstellung eines individuellen Computermodels dieser Hüfte. Ein zentraler Punkt dieser Weiterbildung der Erfindung ist auftretende Kräfte im Gelenk analysiert werden. Hierfür wird ein mathematisches Modell in Form von "Finite Elementen" erstellt. In die Analyse gehen Kräfte beeinflussende Strukturen wie Muskel, Sehnen, Knorpel und Körpergröße und Körpergewicht sowie anatomische Kenngrößen z.B. wie der Caput-collum-Diaphysen-Winkel mit ein.

Grundlage für das Modell können Daten aus den Ultraschallmessungen aus Anspruch 1,2 und 3 unter der Verwendung der Geräte aus Anspruch 5,6 und 7 sein. Weitere Daten werden aus Röntgenbildern, Computertomographischen Aufnahmen und vor allem hochauflösende Kernspinaufnahmen gewonnen. Aus den erhobenen Schnittbildern wird durch Segmentierung ein Knochenmodell von Hüftpfanne und Schenkelhals erstellt. Bei Kernspinaufnahmen kann der bestehende Gelenkknorpel ebenfalls segmentiert und in das Modell übertragen werden. Aus den segmentierten Schnitten wird ein dreidimensionales Modell des Gelenkes aus Finiten Elementen erstellt. In das Modell werden relevante Weichteilstrukturen wie Muskeln, Sehnen und Bänder eingepasst.

Unter Berücksichtigung der an das Gelenk angreifenden Kräfte, erfolgt dann eine Bewegungssimulation des Gelenks. Zum einen werden Bewegungen des alltäglichen Lebens (Gehen, Sitzen, Stehen, Laufen) simuliert und auch Extrembewegungen wie z.B. eine tiefe Abfahrthocke beim Skifahren oder die Swing-Bewegungen beim Golfspielen simuliert.

Weiterhin können Bewegungen, bei denen es zu pathologischem Körperschallereignissen gekommen war, die wie in Anspruch 8 beschrieben aufgezeichnet wurden, simuliert. So kann gezielt nach pathologischen Bewegungen gesucht werden. Die im Gelenk auftretenden Kräfte werden optisch dargstellt, wobei Spitzenbelastungen gesondert hervorgehoben werden.

Ein weiterer Schritt besteht in der Simulation einer geplanten gelenkerhaltenden Operation. Die Operation wird virtuell durch eine Veränderung des Computermodells simuliert. Dieses geänderte Modell wird erneut einer Bewegungsanalyse unterzogen. Die Anpassung des Modells und die nachfolgende Bewegungsanalyse wird so oft wiederholt, bis ein zufriedenstellendes Ergebnis vorliegt und es zu keiner krankhaften Gelenkbelastung mehr kommt.

Abschließend kann das erstellte individuelle Computermodell als Planung für eine Operation in ein Navigationssystem eingespielt werden. Damit steht dem Operateur eine intraoperative Kontrolle zur Verfügung.

Zusammenfassend wird vorstehend ein gravimetrisches Referenzierungssystem und für eine verbesserte Diagnostik ein Rechner unterstütztes Diagnosegerät für eine Standarisierung der Ultraschallmessungen vorgeschlagen. Insgesamt bietet diese Erfindung eine kostengünstige Alternative zu den bisherigen aufwendigen und strahlenbelastenden CT Untersuchungen. Durch eine einfache bauliche Erweiterung und Bildverarbeitende Algorithmen wird die Ultraschalldiagnostik entscheidend erweitert. Durch eine vorgeschlagene Analysesoftware wird die Befundanalyse der Ultraschallmessungen standardisiert und für wenig erfahrene Untersucher zugänglich. Damit steht ein strahlungsfreies Routinemessverfahren für die Hüftdiagnostik zur Verfügung. Ein weiteres Gerät erfasst den in einem Gelenk entstehenden Körperschall, der unter synchroner Registrierung der Stellung der Extremitäten, aufgezeichnet wird. Der aufgezeichnete Körperschall kann zur Analyse auf ein weiteres Analysegerät übertragen werden. Bewegungsabläufe bei denen es zu einem Schallereignis gekommen war, können so nachvollzogen werden. Auf Grundlage von Ultraschallmessungen wird ein dreidimensionales Knochenmodell erstellt. In diesem Modell können Bewegungen analysiert werden. Für eine dynamische Analyse wird unter Verwendung von Kernspindaten ein individuelles anatomisches Modell in Form eines Finite Elemente Modells erstellt. Mit diesem, für jeden Patienten individuell erstelltem Gelenkmodell werden verschiedene Bewegungsabläufe simuliert. Die im Gelenk auftretenden Kräfte werden durch dieses Verfahren analysiert. Eine geplante Gelenk erhaltende Umformungsoperation kann in diesem Modell simuliert und das Operationsergebnis überprüft werden.

List der verwendeten Bezugszeichen:

| | |
|---|---|
| AAS | Winklerfassungssystem |
| ACD | automatische Kontur Erkennung |
| AMS | automatisches Vermessungssystem |
| ASIS | Spina iliaca anterior superior |
| BAS | Knochenanalysesoftware |
| BAT | Batterie |
| BMS | Formmodellierungs-System |
| BRD | Referenzdatenbank für Knochengeometrie |
| C | Knorpelschichtdicke |
| CAM | Nockenwellendeformität |
| D | Hüftkopfdiagonale |
| DR | Daten Recorder |
| E | Hüftkopfäquator längs |
| F | Fußpunkt unter Hüftkopfäquator |
| FC | Oberschenkelknochenkortikales |
| FH | Hüftkopf |
| FHC | Hüftkopfkortikalis |
| FN | Schenkelhals |
| FNC | Schenkelhalskortikalis |
| FNL | Schenkelhalsgrundlinie |
| GL | Horizontale |
| GS1 | Lagesensor 1 |
| GS2 | Lagesensor 2 |
| H1 | mediale Hüftkopfsekante |
| H2 | lateraler Hüftkopfsekante |
| H2 | pathologische laterale Hüftkopfsekante |
| HIP | Hüftgelenk |

OS      Hüftkopf-Hals Offset

P      Pfannenrand

PAT      Patient

PBM      Knochenmodell des Patienten

PEF      Lot durch E auf FNL

PEL      Beckenknochen

PPT      Beckenlagemessgerät Halterung

R      Hüftkopfradius

S      Scheitelpunkt

S'      pathologischer Scheitelpunkt

SD      Körperschallsensor

STUS      Standard Ultraschallgerät

SYM      Symphyse

TR      Datenfunkübertragung

TRU      Datenfunk Modul

UP      Ultraschall-Transducer

UPGS      Ultraschallkopf mit automatischer Lagekorrektur

US      Schallschatten

WAS      Lagesensor mit drahtloser Datenübertragung

WF      Schallebene

$\alpha$      Winkel in GS1 gegenüber GL

$\beta$      Winkel in GS2 gegenüber GL

**Patentansprüche**

1. Bildgebendes Messverfahren unter Verwendung einer Anordnung zur Ultraschalldiagnostik mit einer Ultraschallsende- und Empfangseinheit als Diagnoseverfahren an einem Hüftgelenk, **gekennzeichnet durch** die folgenden Schritte:

     Bestimmung des Hüftkopfdurchmessers;
     Bestimmung des Kopf-Hals-Offsets;
     Vermessung des lateralen Hüftkopfes H2;
     Berechnung der Hüftkopfgeometrie und
     Interpretation der Messwerte bzw. Bestimmung der Exzentrizität.

2. Bildgebendes Messverfahren nach Anspruch 1, **gekennzeichnet durch** eine Standardisierung von Ultraschalluntersuchungen **durch** Verwendung von mindestens zwei gravimetrischen Lagesensoren, oder **dadurch** gekennzeichnet, dass ein Lagesensor zusätzlich an der zu untersuchenden Extremität angebracht wird, um die Extremität frei gegenüber dem Schallkopf bewegen und die relative Winkelabweichung registrieren zu können, vorzugsweise auch über der Zeit, wobei insbesondere bei diesem Messverfahren parallel zu einer Lageänderung auch über der Zeit auftretende akustische Signale bzw. Körperschall aufgenommen und ausgewertet werden können.

3. Bildgebendes Messverfahren nach einem der vorstehenden Ansprüche, **gekennzeichnet durch** die Verwendung mindestens eines gravimetrischen Lagesensors mit drahtloser Datenübertragung.

4. Bildgebendes Messverfahren nach einem der vorstehenden Ansprüche, **gekennzeichnet durch** die Verwendung einer Kombination aus mindestens einem Lagesensor und einer automatischen Lageregelung einer Transducer Einheit, insbesondere in einer geschlossenen Schallkopfeinheit.

5. Bildgebendes Messverfahren nach einem der vorstehenden Ansprüche, **gekennzeichnet durch** die Verwendung einer baulichen Einheit zur Bestimmung und Vermessung einer vorderen Beckenebene.

6. Bildgebendes Messverfahren nach einem der vorstehenden Ansprüche, **dadurch gekennzeichnet, dass** unter Verwendung von Schablonen aus den Messdaten heraus charakteristische Größen zur Beurteilung einer Deformation ermittelt werden, wobei insbesondere auf Bilderkennung basierende Algorithmen automatisch in einer Recheneinheit ablaufen oder **gekennzeichnet durch** die Verwendung einer rechnerunterstützen Datenanalyse von Ultraschallbildern und sonstigen Messergebnissen.

7. Bildgebendes Messverfahren nach einem der vorstehenden Ansprüche, **gekennzeichnet durch** die Erstellung eines dreidimensionalen Knochenmodells aufgrund von Ultraschallbildern und anderen Messgrößen.

8. Bildgebendes Messverfahren nach einem der vorstehenden Ansprüche, **gekennzeichnet durch** die Aufzeichnung von Gelenkgeräuschen **durch** eine mobile Einheit unter Dokumentation der jeweiligen Gelenkstellung.

9. Bildgebendes Messverfahren nach einem der vorstehenden Ansprüche, **gekennzeichnet durch** die Erstellung einer Gelenksimulation zur Analyse pathologischer Gelenkkonstellationen.

10. Vorrichtung zur bildgebenden Ultraschallmessung mit einer Ultraschallsende- und Empfangseinheit, **dadurch gekennzeichnet, dass** eine Anordnung zur bildgebenden Ultraschalldiagnostik durch Kopplung deren Ultraschallsende- und Empfangseinheit in einem Schallkopf (2) um einem Lagesensor (5) derart erweitert ist, dass die mittels Auswertung von Ultraschall-Signalen erhaltenen Messdaten mit Lagedaten der Ultraschallsende- und Empfangseinheit kombiniert sind, wobei der Lagesensor (5) mit der Ultraschallsende-und Empfangseinheit als Schallkopf (2) eine bauliche Einheit bilden, die insbesondere auch zur Bedienung von Hand ausgebildet ist.

11. Vorrichtung nach dem vorhergehenden Anspruch, **dadurch gekennzeichnet, dass** der Lagesensor (5) an der Ultraschallsende- und Empfangseinheit bzw. im Schallkopf (2) zusammen mit einem weiteren, an der zu untersuchenden Extremität angebrachten Lagesensor eine zeitlich parallel auszuwertende Einheit bildet.

12. Vorrichtung nach einem der beiden vorhergehenden Ansprüche, **dadurch gekennzeichnet, dass** als Lagesensor (5) die Technik eines SmartPhones oder die Technik einer Eingabevorrichtung für eine Spielekonsole verwendet und zeitlich parallel zu einer Lage oder Lageänderung über der Zeit auch akustische Signale aufgenommen und ausgewertet werden.

Abb 1.1

Abb. 1.2:

Abb. 1.3:

Abb. 1.4:

Abb 1.5:

Abb. 1.6:

Abb. 1.7:

Abb. 1.8:

Abb.: 2.1

Abb: 2.2:

Abb.: 3

Abb.: 4

Abb.: 5.1:

Abb.: 5.2

Abb.: 6:

Abb.: 7

UP

PAT    WAS

TR

TRU

STUS

BRD

AAS

Algorithmus

ACD

BMS

AMS

NAME: Bauer Mike
DATE: 19700801
ID:   2333442191

Offset =    4,5 mm
Diameter = 55,2mm
Ration =    0,72
esult. CAM  I-3

PBM

BAS

Abb. 8:

TSD

DR

GY

BAT

DR

SD

SD

PAT

HIP

GS + GY

Abb. 9.1:

Abb.9.2:

| | Europäisches Patentamt<br>European Patent Office<br>Office européen des brevets | **EUROPÄISCHER RECHERCHENBERICHT** | **Nummer der Anmeldung**<br>EP 11 15 8350 |
|---|---|---|---|

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | R. GRAF: "Hip sonography. Basic principles and current aspects", DER ORTHOPÄDE, Bd. 26, Nr. 1, 30. Januar 1997 (1997-01-30), Seiten 14-24, XP55002631, ISSN: 0085-4530, DOI: 10.1007/PL00003320 | 1,5-7,9 | INV.<br>A61B8/08<br>A61B5/103 |
| Y | * das ganze Dokument *<br>----- | 2-4,8 | |
| X | WO 2006/068103 A1 (HITACHI MEDICAL CORP [JP]; OGIHARA MAKOTO [JP]; KUBOTA JUN [JP]; SASAK) 29. Juni 2006 (2006-06-29) | 10-12 | |
| Y | * Zusammenfassung *<br>* Absatz [0011] *<br>* Abbildungen 2(a), 2(b), 6(a), 6(b) *<br>----- | 2-4 | |
| Y | DE 10 2007 048595 B3 (RUWISCH DIETMAR [DE]) 26. Februar 2009 (2009-02-26)<br>* Absatz [0014] *<br>----- | 8 | |
| X | D LEOTTA: "Performance of a miniature magnetic position sensor for three-dimensional ultrasound imaging", ULTRASOUND IN MEDICINE & BIOLOGY, Bd. 23, Nr. 4, 1. Januar 1997 (1997-01-01), Seiten 597-609, XP55002484, ISSN: 0301-5629, DOI: 10.1016/S0301-5629(97)00043-4<br>* das ganze Dokument *<br>-----<br><div align="center">-/--</div> | 10,12 | RECHERCHIERTE SACHGEBIETE (IPC)<br><br>A61B |

Der vorliegende Recherchenbericht wurde für alle Patentansprüche erstellt

| Recherchenort | Abschlußdatum der Recherche | Prüfer |
|---|---|---|
| München | 13. Juli 2011 | Willig, Hendrik |

EPO FORM 1503 03.82 (P04C03)

| Europäisches Patentamt<br>European Patent Office<br>Office européen des brevets | **EUROPÄISCHER RECHERCHENBERICHT** | **Nummer der Anmeldung**<br>EP 11 15 8350 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | HUGHES S W ET AL: "VOLUME ESTIMATION FROM MULTIPLANAR 2D ULTRASOUND IMAGES USING A REMOTE ELECTROMAGNETIC POSITION AND ORIENTATION SENSOR",<br>ULTRASOUND IN MEDICINE AND BIOLOGY, NEW YORK, NY, US,<br>Bd. 22, Nr. 5, 1. Januar 1996 (1996-01-01)<br>, Seiten 561-572, XP002060896,<br>ISSN: 0301-5629, DOI:<br>10.1016/0301-5629(96)00022-1<br>* das ganze Dokument *<br>----- | 10,12 | |
| X | LINDSETH F ET AL: "Probe calibration for freehand 3-D ultrasound",<br>ULTRASOUND IN MEDICINE AND BIOLOGY, NEW YORK, NY, US,<br>Bd. 29, Nr. 11,<br>1. November 2003 (2003-11-01), Seiten 1607-1623, XP004477173,<br>ISSN: 0301-5629, DOI:<br>10.1016/S0301-5629(03)01012-3<br>* das ganze Dokument *<br>----- | 10,12 | **RECHERCHIERTE SACHGEBIETE (IPC)** |

Der vorliegende Recherchenbericht wurde für alle Patentansprüche erstellt

| Recherchenort | Abschlußdatum der Recherche | Prüfer |
|---|---|---|
| München | 13. Juli 2011 | Willig, Hendrik |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
..............................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 11 15 8350

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-07-2011

| Im Recherchenbericht angeführtes Patentdokument | Datum der Veröffentlichung | Mitglied(er) der Patentfamilie | Datum der Veröffentlichung |
|---|---|---|---|
| WO 2006068103 A1 | 29-06-2006 | KEINE | |
| DE 102007048595 B3 | 26-02-2009 | EP 2197353 A1<br>WO 2009050089 A1<br>US 2010222675 A1 | 23-06-2010<br>23-04-2009<br>02-09-2010 |

EPO FORM P0461

Für nähere Einzelheiten zu diesem Anhang : siehe Amtsblatt des Europäischen Patentamts, Nr.12/82